# EUROPEAN PATENT APPLICATION

(11) **EP 0 568 500 A1**
(43) Date of publication of application: **03.11.1993**
(21) Application number: 93830121.5
(22) Date of filing: 26.03.1993
(51) Int. Cl.: A61K 39/395, C12P 21/08, G01N 33/577, C07K 15/00

(54) **Anti-idiotype monoclonal antibodies directed against anti-TNF antibodies**

(30) Priority: 27.03.1992 IT RM920223
(71) Applicant: MINISTERO DELL' UNIVERSITA' E DELLA RICERCA SCIENTIFICA E TECNOLOGICA, I-00196 Roma (IT)
(72) Inventor: Corti, Angelo, I-20131 Milano (IT)
(74) Representative: de Simone, Domenico

(57) **Abstract**

Anti-idiotype monoclonal antibodies are disclosed, said antibodies being capable of recognizing and binding at least oneidiotope of an anti-TNF antibody.

## Description

This invention relates to anti-idiotype monoclonal antibodies directed against anti-TNF antibodies.

More particularly, this invention relates to the identification of anti-idiotype antibodies directed against anti-tumoral necrosis factorα(TNFαor cachectin) antibodies to be employed in diagnostic and in clinical uses.

As regards the scope of this invention, antibodies are protein molecules which are made up of light and heavy chains, and which are capable of recognizing and then binding some particular sites, that are called epitopes and are present on the antigen molecule. The three-dimensional structure of the antigen-binding site, which is called the paratope, is made up of six hypervariable regions, three on the heavy chain and three on the light one; such regions being coded by the genes of the immunoglobulin variable regions (V). The hypervariable regions of each antibody molecule are made up of unique sequences which are potentially immunogenic also in syngenic animals. The set of antigenic determinants (idiotopes), which are made up of the V regions of a particular immunoglobulin, is defined as an idiotype. Accordingly, it is possible to immunize syngenic animals with monoclonal or polyclonal antibodies (Ab1) directed against particular antigens, and to generate anti-antibodies, which are called anti-idiotype antibodies (Ab2), that are capable of recognising the immunogen Ab1 selectively. Various types of Ab2 exist, which can be classified according to the topographic relation between the recognized idiotopes and the site of combination with the antigen (the paratope) of the Ab1. In particular, the Ab2ₛ which can recognize an idiotope external to the paratope and hence are incapable of inhibiting the binding of the antigen with the Ab1, are defined as alpha-Ab2; the antibodies that on the contrary bind a site internal to the paratope and hence inhibit the binding of the antigen with the Ab1 efficiently, are defined as beta-Ab2. There is a further class of Ab2ₛ , that is defined as the gamma-Ab2 and shows intermediate characteristics: such antibodies, though they bind a site external to the paratope, are capable of inhibiting sterically the binding of the antigen with the Ab1, but they are not capable of mimicking the molecular and the biological properties of the antigen itself.

The anti-idiotype monoclonal antibodies have been employed advantageously as molecular substitutes of the antigens in the development of immunological tests and of chromatographic systems for analysis and purification of immuno-reactive fractions of radiolabelled antibodies, in the preparation of vaccines against infectious diseases as well as in the treatment of neoplastic diseases (Kohler H. et al. Methods in Enzymol. 98, 3-35, 1989). The advantages in connection with the employment of anti-Idiotype antibodies are evident when the antigen is available in low amounts or is unstable, when it cannot be employed as such for therapeutic or immunogenic purposes, because of its toxicity or pathogenicity, or when it is desired to mimick just some effects of the antigen.

TNF is a cytokine which is mainly secreted by activated macrophages, and has been originally identified in the serum of mice infected with the Bacillus "Calmètte Guerin" (BCG) because of its ability of inducing haemorrhagic necrosis of some tumors in animal experimental models.

Successively it has been discovered that TNF is one of the main mediators of inflammation. In some particular occasions of acute and/or chronic production, TNF can give rise to pathological states like the septic shock, cerebral malaria and lipid dismetabolism (cachexia) typical of patients suffering from chronic infectious and neoplastic pathologies. Moreover, it has been shown that TNF participates together with other cytokines and lymphokines in immunoregulation, angiogenesis, cell growth and differentiation processes (Beutler B. and Cerami A. 1989 Ann. Rev. Immunol. 7, 625-655).

It is believed that many, if not all, of the effects produced by TNF are mediated by receptors which are present on the membrane of target cells. Two different receptor types have been purified and characterized at the present time (Smith C.A. et al. 1990 Science 248, 1019-1023; Schall T.J. et al. 1990 Cell 61, 361-370). Moreover, soluble forms corresponding to the extracellular portion of both receptors, which forms are endowed with a powerful inhibition activity towards TNF,have been purified from urines of feverish patients suffering from chronic renal insufficiency, or from the serum of patients suffering from neoplastic diseases (Nophar Y. et al. Embo J. 9, 3269-3278).

In some pathological situations, like septic shock (Waage et al., 1987, Lancet i; 355), rejection of renal transplants (Maury et al., 1987, J. Exp. Med. 166, 1132), parasitic infections (Scuderi et al., 1986, Lancet ii, 1364), and various neoplastic diseases (Balkwill et al., 1987, Lancet ii, 1229) the TNF plasmatic levels increase at a remarkable degree.

Accordingly, it is evident that there is the need for the development of compounds capable of decreasing the toxic effects induced by endogenous TNF under hyperproduction conditions, through the neutralization of the TNF-receptor bond.

Among the anti-TNF antibodies, those capable od exerting a neutralizing action are particularly useful, which are capable of recognizing TNF epitopes sterically close to or coinciding with the binding sites with the TNF receptors, so as to prevent the binding TNF-receptor and hence to stop the biological effects. The antibody Mab78 (GB Patent Application No. 9028123.1; Barbanti et al., "2 Int. Congress on Thrauma, Shock and Sepsis", 1991, Munich, Germany) belongs to that category.

The Author of this invention has isolated anti-idiotype monoclonal antibodies directed against anti-TNF antibodies, to be employed as molecular substitutes of TNF in all cases in which said TNF is of short supply or is unstable, or when it cannot be employed as such for therapeutical or immunogenic purposes, because of its toxicity or pathogenicity or when it is desired to mimick just some of its effects. More particularly, such antibodies are employed for developing immunological assays and chromatographic systems for analysis and purification of immunoreactive fractions of anti-TNF antibodies, for the preparation of immunogens for preventing the pathological symptoms induced by TNF.

Accordingly, the object of this invention consists in anti-Idiotype monoclonal antibodies capable of recognizing and binding at least one idiotope of an anti-TNF antibody.

According to a preferred embodiment of the invention, said idiotope is included in the paratope of the anti-TNF antibody; preferably, said anti-idiotype monoclonal antibodies are capable of inhibiting the binding between TNF and the anti-TNF antibody; and even more preferably, they are derived from immunization of mice, and again more preferably they can be obtained from the Mab1G3 hybridoma line deposited with the DSM, N. ACC2032.

According to an alternative embodiment of this invention, said idiotope is external to the paratope of the anti-TNF antibody; preferably said anti-idiotype monoclonal antibodies are capable of interfering with the binding between TNF and the anti-TNF antibody, and alternatively they are incapable of interfering with the binding between TNF and the anti-TNF antibody; preferably said anti-idiotype monoclonal antibodies are derived from immunization of mice, and even more preferably they are obtainable from the Mab9F1 hybridoma line deposited with the DSM, N. ACC2031.

It is a further object of this invention the employment of anti-idiotype monoclonal antibodies against anti-TNF antibodies for the preparation of immunological diagnostic kits, of chromatografic systems and of pharmaceutical compositions.

This invention will be now disclosed by means of explanatory examples of the same which have no limitative purposes, and in which reference will be made to the following figures:
- Figure 1 represents an anti-Mab78 antibody assay from sera of animals immunized with Mab78 conjugates; the sera are assayed by means of direct ELISA assay (upper frame) and the competitive ELISA assay (the lower frame);
- Figure 2 shows a solid phase binding assay of anti-Id antibodies, which are labelled with peroxidase, to mouse IgG;
- Figure 3 shows a competitive binding assay of the Mab1G3 anti-Id antibodies and the Mab9F1 (Ab2) anti-Id antibodies with the Mab78 antibody;
- Figure 4 represents a competitive binding assay of the TNF to the Mab78 with Mab1G3 and Mab9F1, determined by means of various detection systems;
- Figure 5 shows a competitive binding assay between the TNF and the Mab1G3 or Mab9F1 anti-Id antibodies for the binding with Mab78;
- Figure 6 represents an assay of the inhibition of the activity that neutralizes the cytotoxicity of TNF of Mab78 with Mab1G3 and Mab9F1;

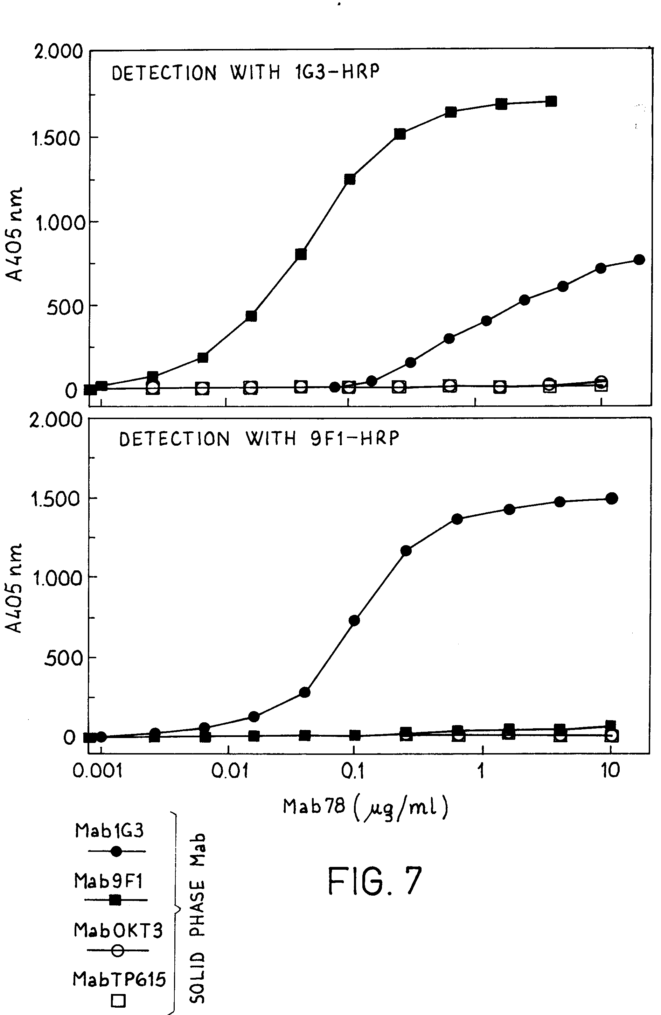

- Figure 7 represents an ELISA detection assay of bivalent Mab78s, employing anti-Id antibodies.

### Example 1

### Preparation of anti Mab78 anti-idiotype antibodies

The Mab78 antibody (ECACC 90110707 deposited on 7-11-90; GB Patent Application Nb.9028123.1; Barbanti et al., "2 Int. Congress on Thrauma, Shock and Sepsis", 1991, Munich, Germany) has been conjugated to bovine seric albumin (BSA), cationized according to the directions of the producer (SuperCarrier, Pierce). 500 µl of a Mab78 solution, 3 mg/ml in PBS, has been mixed with 75 µl of a haemocyanin (KLH) or of BSA, 20 mg/ml in PBS, and with 20 µl of glutaraldehyde at 2.5 % concentration. The solution has been incubated for 2 hours at room temperature, mixed with 50 µl of 1 M lysine, and then the solution has been further incubated for 4 hours at room temperature. The products have been dialyzed against PBS, then diluted to 1 ml total volume and stored at -20°C. The conjugation efficiency has been monitored by SDS electrophoresis, and it was always found to be larger than 90 %.

BALB/c (Charles River) female mice were immunized through intraperitoneal route with 100 µg of Mab78-carrier conjugates, 1/1 emulsified with complete Freund adjuvant (CFA). The animals were given a booster at an interval of two weeks, with the same dose of conjugate with incomplete Freund's adjuvant once, or in PBS for three times. Three days after the latest booster, mice were sacrified, the spleen cells were isolated and fused with NSO myeloma cells in agreement with standard procedures (Galfrè and Milstein, 1981, Methods in Enzymol. 73, 1).

A probing of the culture medium in which hybridomas have been grown has been carried out employing a direct ELISA assay and a competitive ELISA assay as disclosed in the following. The isotype determination has been performed employing the "Mouse Typer Isotyping Kit" in agreement with the directions of the producer (Biorad, Richmond, CA 94804). The Mab9F1 (IgG1) and Mab1G3 (IgG2a) antibodies were purified from the culture media of cells by affinity chromatography on protein G agarose (Schmidt, 1989, J. Biotechnol., 11, 235-252). The final products were shown to be 90 % pure, as it was determined through electrophoresis performed in the presence of SDS.

### Example 2

### Preparation of reagents labelled with biotin or with peroxidase (HRP)

Both antibodies and TNF were biotinylated employing standard procedures, antibodies-HRP conjugates were prepared in the following way: 100 µl of each one of the purified monoclonal antibodies, 3 mg/ml in PBS, were mixed with 100 µl of an HRP solution, 10 mg/ml in 0.1 M sodium phosphate buffer, pH 6.5, and with 10 µl of 1 % glutaraldehyde. The solutions were incubated for 4 hours at room temperature and then they were mixed with 20 µl of a 1 M lysine solution. After 1 hour incubation, the products were dialyzed against a phosphate buffer and they were stored at -20°C.

### Example 3

### Determination of anti-idiotype (anti-Id) antibodies by means of direct ELISA

PVC microtitration plates were treated for one night with 40 µg/ml of a solution of goat anti-mouse (GAM-IgG) antibodies, 50 µl/well, in PBS at 4°C and then the plates were blocked with 200 µl of PBS containing 3 % BSA for two hours at 37°C. After various washing with PBS-T (PBS with 0.05 % Tween 20) each well was filled with 50 µl of samples and incubated for 1 hour at 37°C. The free binding sites were blocked with 50 µl/well of 10 % mouse serum in PBS-BT (PB-T with 2 % BSA) for 1 hour at 37°C. 50 µl of biotinylated Mab78, 1/500 in PBS-BTN (PBS-BT with 1 % goat serum) was added to each well and incubated for 1 hour at 37°C. After further washing with PBS-T, the plates were incubated with 50 µl/well of streptavidin conjugated to HRP (HRP-STV) 1: 2000 in PBS-BT for 1 hour at 37°C, then the plates were washed again and incubated with 100 µl of a chromogenic ABTS solution (Kirkegaard & Perry Lab. Inc.) for 30 minutes at 37°C. The absorbances at 405 nm of each well were then determined on a 2550 EIA model reader (Biorad).

### Example 4

### Determination of anti-Id antibodies by means of competitive ELISA

The microtitration plates were treated for one night with 1 µg/ml of Mab78, 50 µl/well, in PBS at 4°C and blocked with 200 µl of PBS containing 3 % BSA for 2 hours at 37°C. After washing with PBS-T, samples of anti-Id antibodies (50 µl) were added to each well and then the samples were incubated for 1 hour at 37°C. The plates were then washed as already disclosed, then filled with 50 µl/well of TNF biotinylated, 1/1000 in PBS-BT, and then the plates were incubated for 1 hour at 37°C. The amount of biotinylated protein bonded to each well was estimated with STV-HRP as disclosed for the direct ELISA assay of anti-Id antibodies.

### Example 5

### Generation of anti-Id antibodies

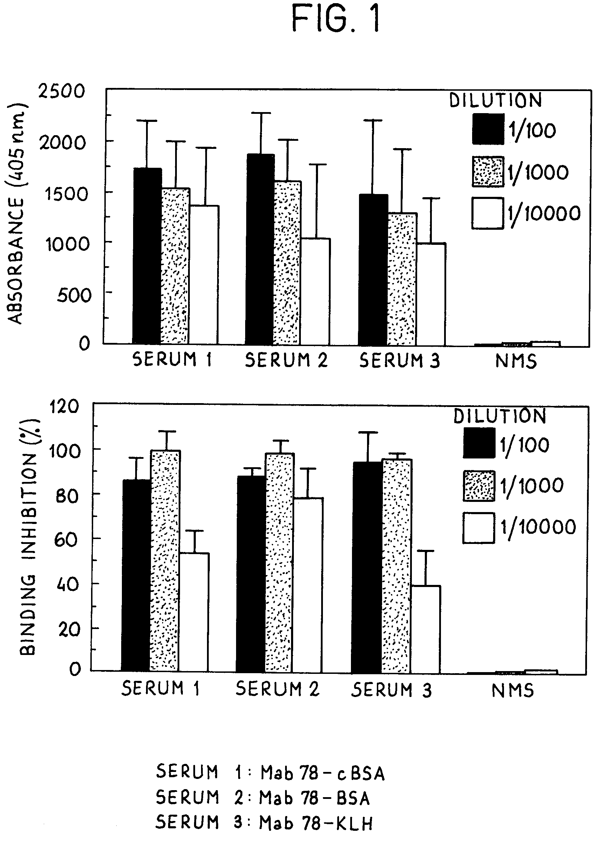

The presence of anti-Mab78 antibodies in the serum of nine Balb/c mice immunized with Mab78-cBSA, Mab78-BSA or Mab78-KLH conjugates has been estimated by means of a direct binding assay with Mab78 (direct ELISA) and with competitive binding with biotinylated TNF (competitive ELISA). As shown in Figure 1 (the upper frame), all three conjugates are efficiently capable of inducing a good anti-Mab78 immune response, as estimated by the direct ELISA assay. Moreover, all sera were capable of inhibiting in a similar way the binding of TNF biotinylated with Mab78 (the lower frame), suggesting that anti-idiotype antibodies have been induced by immunization. The spleen cells of a mouse that had been immunized with Mab78 KLH were fused with NSO myeloma cells in order to generate hybridomas. From a fusion experiment, 53 clones out of about 800 clones that were growing within the wells, turned out to be positive by a direct ELISA assay. However, just 8 supernatants turned out to be capable of partially inhibiting the TNF biotinylated bonding to Mab78. Two clones belonging to such group, defined as Mab1G3 (DSM N. ACC2032) and Mab9F1 (DSM N. ACC2031) were selected because of their stability after repeated subcloning experiments. Isotyping experiments showed that Mab1G3 and MabF1 were IgG2a (k) and IgG1 (k) respectively.

### Example 6

### Specificity of the anti-Id-antibodies for the immunizing Mab78

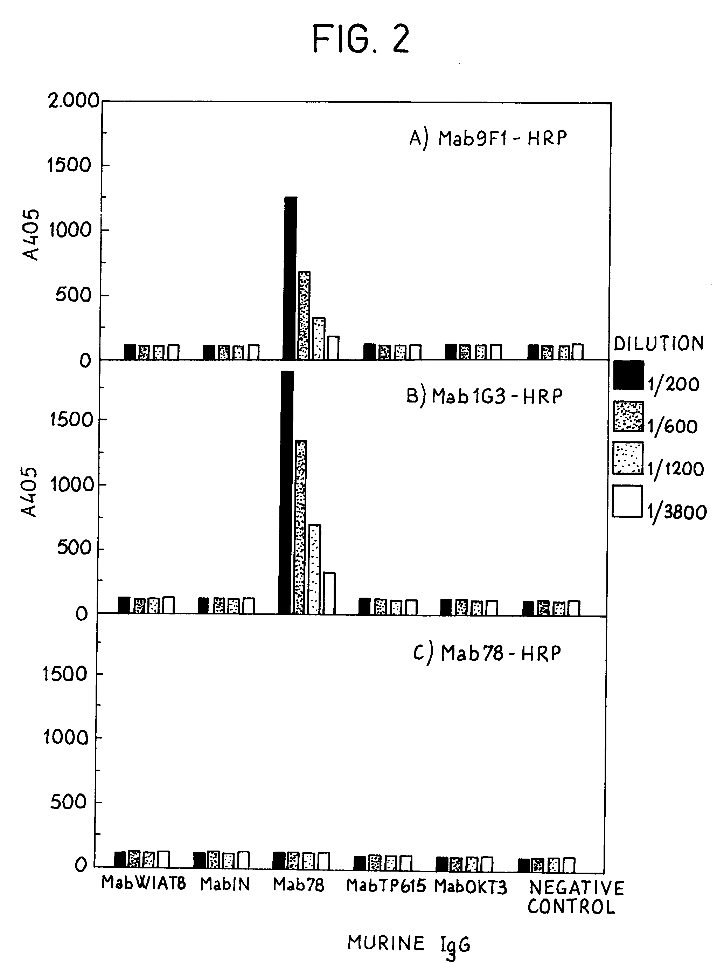

In order to determine the anti-Id nature of the Mab1G3 and Mab9F1 antibodies, the binding ability of both antibodies to Mab78 or to non-specific immunoglobulins was examined. As shown in Figure 2 (the frames A and B), Mab1G3 and Mab9F1 labelled with HRP are capable to bind Mab78 (IgG1 k) but not other antibodies, both of the same and of a different antigenic specificity and Isotype, MabWIAT4 (anti-TNF, IgG1 k), MabIN (anti-TNF, IgG1 k), MabTP615 (anti-antigen associated to the high molecular weight melanoma IgG1 k), and Mab0KT3 (anti-CD3, IgG2a k) included (Figure 2). The binding is specific, as Mab78-HRP is incapable of binding said antibodies (the frame C), whereas it is capable of solid phase binding Mab1G3 and Mab9F1 efficiently, so confirming the anti-Id nature of Mab1G3 and Mab9F1.

### Example 7

### Idiotope topography of Mab1G3 and Mab9F1

The topographical relationships between the idiotopes recognized by Mab1G3 and Mab9F1 have been studied with binding competition experiment to the Mab78.

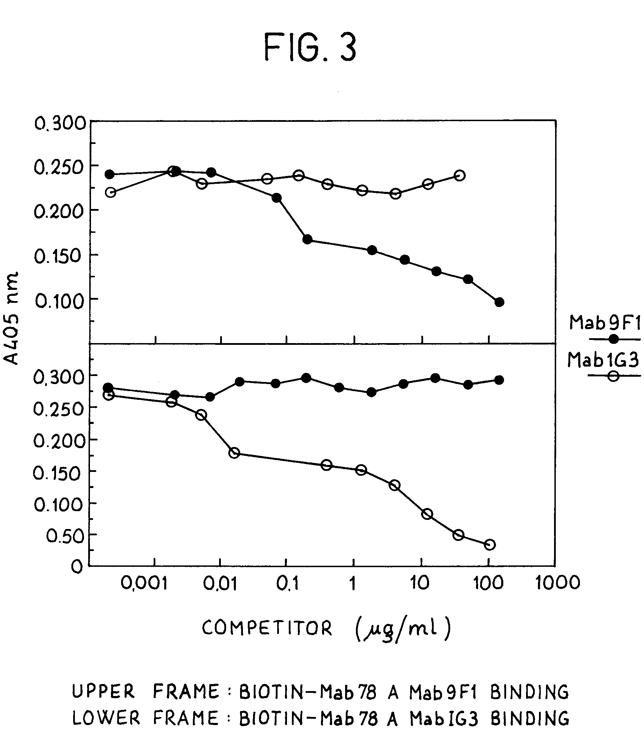

As shown in Figure 3, the solid phase binding of Mab78 to Mab9F1 is efficiently inhibited by previous incubation with soluble Mab9F1, but not with Mab1G3 (see the upper frame). Moreover, the binding of biotinylated Mab78 to Mab1G3 in the solid phase is efficiently inhibited by incubation with soluble Mab1G3 but not by Mab9F1 (see the lower frame). Such results point out that Mab1G3 and Mab9F1 recognize two idiotopes which are sterically distinguished on Mab78.

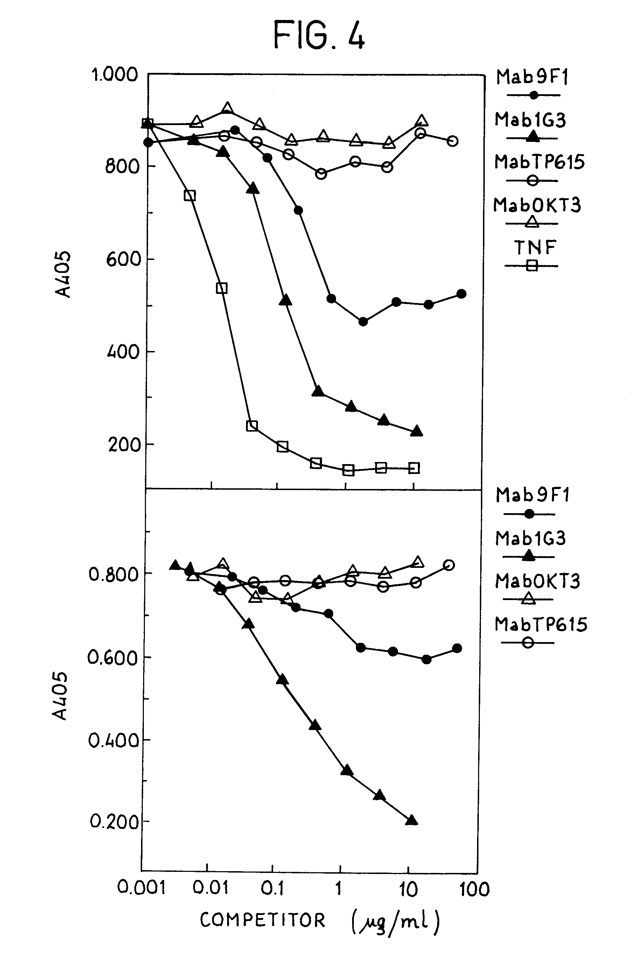

Accordingly, the topographic relationship between the idiotopes and the paratope on Mab78 has been investigated. To that aim, the competition between TNF and anti-Id antibodies for the Mab78 binding in solid phase has been examined in two different analytical systems which are based on: a) competitive binding with TNF labelled with biotin and evidence obtained by means of streptavidin HRP; b) competitive binding with unlabelled TNF and evidence obtained with an antiserum of rabbit anti-TNF and GAR-HRP. As shown in Figure 4, Mab1G3 is capable of inhibiting efficiently the binding both of biotin-labelled TNF and of unlabelled TNF, whereas Mab9F1 is capable of inhibiting the binding just partially, from about 25 to 50 %, in a way that depends on the detection system.

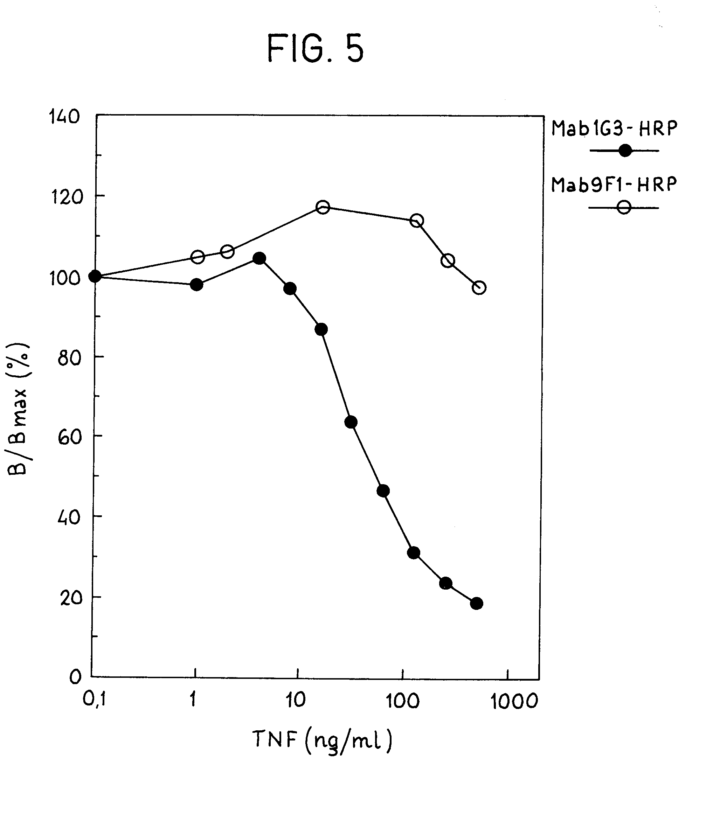

In order to establish whether the observed inhibition is related to the steric interference of the TNF bond or of the reagents that evidence TNF, the competition between TNF and anti-Id antibodies labelled with HRP for the binding to Mab78 has been successively examined. As shown in Figure 5, the TNF is efficiently capable of inhibiting the binding of Mab1G3-HRP to Mab78, with a dose/response curve in the range between 5 and 500 ng/ml. On the contrary, TNF is incapable of inhibiting the binding of Mab9F1-HRP, even at saturation concentrations (500 ng/ml, as checked previously with TNF biotin).

The idiotope topography has been further investigated in a liquid phase system. To that aim, a cytolytic assay on LM cells has been carried out to evaluate the capability of anti-Id antibodies of inhibiting the neutralizing activity in Mab78s on cytotoxicity induced by TNF. As shown in Figure 5, the neutralizing effect of Mab78 can be completely inhibited by Mab1G3. By contrast, no inhibition has been observed with Mab9F1.

Such results point out that the Mab1G3 antibody binds an idiotope which is close to the combination site of the antigen on Mab78 (paratope) or that overlaps such site, whereas the Mab9F1 antibody binds an idiotope which is localized externally to the paratope.

### Example 8

### Determination of Mab78 by means of ELISA with anti-Id antibodies

Anti-Id antibodies have been employed for developing immunological assays which are Mab78 specific.

To that aim, the capability of the Mab78 antibody to form molecular "sandwiches" with anti-Id antibodies in various ELISA systems has been assayed with unlabelled anti-Id antibodies in solid phase and with anti-Id antibodies labelled with HRT by means of evidencing reagents.

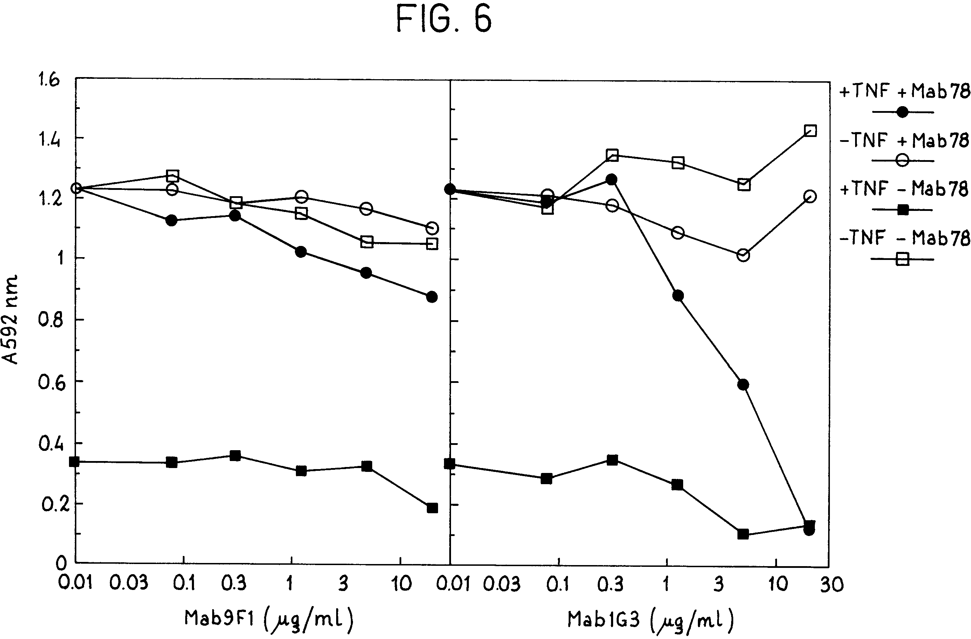

As shown in Figure 6, Mab78 is capable of forming such molecular "sandwiches" in a range between a) 0.02 and 2 µg/ ml with Mab9F1 and Mab1G3-HRP, and b) 0.3 and 20 µg/ml with Mab1G3 and Mab1G3-HRP and c) 0.01 and 5 µg/ml with Mab1G3 and Mab9F1-HRP. The binding is specific, as no binding is evidenced when non-specific IgGs have been employed for coating the plates instead of Mab1G3 or Mab9F1. As a good dose/response curve has been obtained with Mab1G3-coated and with Mab1G3-HRP -coated plates, in the detection phase, such assay can be employed for evidencing divalent Mab78s in antibody conjugates. Moreover, the sandwich complexes of Mab78 with Mab9F1 and Mab9F1-HRP turned out not to be evidenceable, possibly because of the fact that Mab78 binds preferentially with both the Mab9F1 sites in solid phase.

### Example 9

### Development of an analytical procedure for the determination of divalent antibodies (Mab78).

A difunctional ELISA assay for evidencing the divalent Mab78 antibody has been carried out as follows: PVC microtitration plates have been incubated for one night at 4°C, with 10 µg/ml of a solution of Mab1G3 in PBS (50 µl/well). The plates have been washed with PBS and further incubated for 2 hours with 200 µl of 3 % BSA in PBS. After washing with PBS, each well has been filled with Mab78 samples or with a standard, in triplicate, 50 µl/well such samples being already diluted with PBS-BT. After one hour incubation at 37°C, the plates have been washed eight times with PBS-BT and then filled with 50 µl/well of Mab1G3-HRP (1/1OOO and 1/500 respectively in PBS-BT of a stock solution). The plates were then incubated again for two hours at 37°C and washed with PBS-T. The chromogenic reaction and the determinations of absorbance have been carried out as disclosed for the anti-Id direct ELISA assay. The concentrations of divalent Mab78s have been calculated by extrapolating the absorbance values on the calibration curves prepared with standard Mab78 solutions.

## Claims

1. Anti-idiotype monoclonal antibodies capable of recognizing and binding at least one idiotope of an anti-TNf antibody.

2. Anti-idiotype monoclonal antibodies according to claim 1, which are derived by the immunization of animals belonging to the murine species.

3. Anti-idiotype monoclonal antibodies according to any one of the preceding claims in which said idiotope is included in the paratope of the anti-TNf antibody.

4. Anti-idiotype monoclonal antibodies according to claim 3 capable of inhibiting the binding between TNf and anti-TNf antibody.

5. Anti-idiotype monoclonal antibodies according to claim 4, which can be obtained from the Mab1G3 hybridoma line deposited with the DSM N. ACC2032.

6. Anti-idiotype monoclonal antibodies according to any one of the claims 1 or 2 in which said idiotope is external to the paratope of the anti-TNf antibody.

7. Anti-idiotype monoclonal antibodies according to claim 6 capable of interfering with the binding between TNf and anti-TNf antibody.

8. Anti-idiotype monoclonal antibodies according to claim 6 which are incapable of interfering with the binding between TNf and anti-TNf antibody.

9. Anti-idiotype monoclonal antibodies according to claim 8 which can be obtained from the Mab9f1 hybridoma line deposited with the DSM N. ACC2031.

10. Use of anti-idiotype monoclonal antibodies against anti-TNf antibodies according to any one of the preceding claims for the preparation of immunological diagnostic kits.

11. Use of anti-idiotype monoclonal antibodies against anti-TNF antibodies according to any one of the preceding claims 1-9 for the preparation of chromatographic systems.

12. Use of anti-idiotype monoclonal antibodies against anti-TNF antibodies according to any one of the preceding claims 1-9 for the preparation of pharmaceutical compositions.
